(19) Europäisches Patentamt
European Patent Office
Office européen des brevets

(11) Numéro de publication: **0 059 655**
**B1**

# (12) FASCICULE DE BREVET EUROPEEN

(45) Date de publication du fascicule du brevet: **24.07.85**

(51) Int. Cl.⁴: **C 07 D 313/04**, C 07 C 179/10

(21) Numéro de dépôt: **82400229.9**

(22) Date de dépôt: **09.02.82**

(54) **Procédé d'obtention de l'epsilon-caprolactone.**

(30) Priorité: **20.02.81 FR 8103374**

(43) Date de publication de la demande: **08.09.82 Bulletin 82/36**

(45) Mention de la délivrance du brevet: **24.07.85 Bulletin 85/30**

(84) Etats contractants désignés: **AT BE CH DE FR GB IT LI NL SE**

(56) Documents cités:
**EP - A - 0 004 407**
**EP - A - 0 020 952**
**EP - A - 0 022 396**
**EP - A - 0 025 381**
**BE - A - 691 391**
**FR - A - 1 490 173**
**FR - A - 1 531 053**
**FR - A - 2 101 985**

**Gmelin Erg. Werk, Bd. 28, p. 15 (1975)**
**C.A. 63/9432**
**C.A. 65/16112**
**C.A. 52/5187**
**Gmelin, Bd. 28, Teil 7, p. 21 (1975)**

**Le dossier contient des informations techniques**

(73) Titulaire: **ATOCHEM, 12/16, allée des Vosges, F-92400 Courbevoie (FR)**

(72) Inventeur: **Lecoq, Jean-Claude, 10 A, rue Josserand, F-69630 Chaponost (FR)**
Inventeur: **Pralus, Michèle, 3, rue des Gosses, F-69450 Saint-Cyr au Mont d'Or (FR)**
Inventeur: **Schirmann, Jean-Pierre, 49, chemin de la Glacière, F-69600 Oullins (FR)**

(74) Mandataire: **Rochet, Michel et al, ATOCHEM Département Propriété Industrielle Cédex 22, F-92091 Paris la Défense (FR)**

(56) Documents cités: (suite)
**présentées postérieurement au dépôt de la demande et ne figurant pas dans le présent fascicule.**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 059 655 B1

**Description**

La présente invention concerne un procédé de préparation de l'ε-caprolactone par oxydation de la cyclohexanone au moyen d'une solution brute d'acide percarboxylique.

Il est bien connu que les cétones peuvent être oxydées en esters par les acides percarboxyliques, selon la réaction générale:

$$R_1\text{–CO–}R_2 \xrightarrow{R\text{–}CO_3H} R_1\text{–CO–O–}R_2$$

Cette réaction, connue sous le nom de réaction de Baeyer-Villiger, a fait l'objet d'un grand nombre de publications depuis se découverte en 1899. Elle fait intervenir un complexe intermédiaire peroxydique, qui se réarrange selon un processus ionique pour donner l'ester.

Une application importante de cette réaction générale est la préparation des lactones à partir des cétones cycliques:

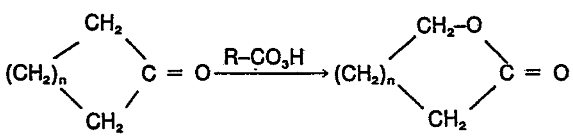


Dans le cas où n = 3, la cétone cyclique est la cyclohexanone, et son oxydation par l'acide percarboxylique conduit à l'ε-caprolactone.

On sait que la stabilité de l'ε-caprolactone est faible en milieu acide. Si l'on veut donc obtenir cette lactone avec de bons rendements directement à partir de la cyclohexanone par la réaction de Baeyer-Villiger, il est essentiel que la solution d'acide percarboxylique utilisée présente l'acidité la plus faible possible. Le problème se ramène donc avant tout au choix de la méthode de préparation de l'acide percarboxylique.

Une première solution préconisée par l'art antérieur consiste à utiliser de l'acide peracétique, préparé par oxydation de l'acétaldéhyde par l'oxygène. L'inconvénient majeur de ce procédé est qu'il faut pouvoir valoriser l'acide acétique coproduit dans la réaction d'oxydation de la cyclohexanone.

Une autre solution de l'art antérieur consiste à préparer l'acide percarboxylique par réaction d'un acide carboxylique avec le peroxyde d'hydrogène. Ce type de procédé a l'avantage de permettre le recyclage de l'acide carboxylique après oxydation de la cyclohexanone.

Cependant les solutions d'acide percarboxylique, obtenues par action du peroxyde d'hydrogène sur un acide carboxylique selon les méthodes connues, présentent toutes une forte acidité, due soit au fait que l'on utilise comme acide carboxylique un acide fort, comme l'acide formique, soit au fait que l'on catalyse la réaction de formation de l'acide percarboxylique par un catalyseur acide fort, tel que l'acide sulfurique.

L'oxydation de la cyclohexanone par des solutions d'acide percarboxylique obtenues selon ces méthodes nécessite la neutralisation du catalyseur acide fort, si l'on veut éviter la solvolyse de la caprolactone et la formation de nombreux sous-produits. On observe alors la précipitation de sels minéraux, difficiles à séparer du milieu, et qui sont souvent eux-mêmes des catalyseurs de dégradation de la caprolactone. Pour éviter ces inconvénients, on a bien proposé, comme dans le brevet belge n° 540 412 et dans le brevet britannique n° 776 758, d'utiliser, comme catalyseur de formation de l'acide percarboxylique, une résine échangeuse d'ions porteuse de groupements acide fort. Ce catalyseur est très efficace et facile à séparer, mais il présente un danger, du fait de la fixation et de l'accumulation sur la résine d'ions métalliques lourds, tels que les ions $Fe^{3+}$, toujours présents, même à faible dose, dans les produits organiques, et connus pour être des catalyseurs de décomposition violente des acides percarboxyliques.

Une autre solution consiste à mettre en œuvre un acide percarboxylique pur, obtenu par distillation, mais ce procédé présente lui aussi des dangers évidents.

«Enfin la demande de brevet européen n° 22 396 au nom de la demanderesse décrit un procédé de préparation d'acide percarboxylique qui consiste à effectuer la réaction du peroxyde d'hydrogène sur un acide carboxylique aliphatique miscible à l'eau en présence d'un solvant susceptible de former un azéotrope avec l'eau afin de pouvoir continuellement éliminer l'eau hors du milieu réactionnel, et en présence d'un oxyde métalloidique comme catalyseur.

La demanderesse vient de découvrir que l'on peut obtenir, avec de bons rendements, des solutions stables d'ε-caprolactone, par oxydation de la cyclohexanone au moyen de solutions brutes d'acide percarboxylique obtenues selon le procédé décrit dans sa demande de brevet français n° 2 464 947, déposée le 7 septembre 1979, et qui consiste à faire réagir le peroxyde d'hydrogène avec un acide carboxylique, en présence d'un acide borique comme catalyseur, et en éliminant continuellement l'eau en cours de réaction par entraînement azéotropique. Ce procédé évite tous les inconvénients liés à la neutralisation des catalyseurs acides forts.

Les acides percarboxyliques mis en œuvre dans l'oxydation de la cyclohexanone selon l'invention contiennent de 2 à 4 atomes de carbone. Les acides peracétique et perpropionique sont préférés.

Les solutions brutes d'acide percarboxylique utilisées selon l'invention sont des solutions homogènes du peracide dans l'acide carboxylique correspondant, et contiennent en outre éventuellement des solvants organiques compatibles avec le peracide et miscibles avec lui. Il est particulièrement avantageux d'utiliser comme solvant organique le solvant qui a été utilisé comme entraîneur azéotropique de l'eau au cours de la préparation de l'acide percarboxylique.

Selon le peracide mis en œuvre, sa proportion dans la solution brute servant à l'oxydation de la cyclohexanone pourra aller de 5 à 40% en poids.

La solution brute d'acide percarboxylique peut également contenir de petites quantités de peroxyde d'hydrogène n'ayant pas réagi avec l'acide

carboxylique, par exemple jusqu'à 0,1 mole de peroxyde d'hydrogène pour 100 g de solution brute.

La réaction d'oxydation de la cyclohexanone par la solution brute de peracide est réalisée de préférence à la pression atmosphérique, mais peut tout aussi bien être conduite à des pressions inférieures ou supérieures. La température de réaction est comprise entre 20 et 120 °C, et de préférence entre 40 et 80 °C.

Le rapport molaire de la cyclohexanone mise en œuvre à l'acide percarboxylique est compris entre 1 et 5, et de préférence entre 1 et 1,5.

La réaction peut être réalisée soit en discontinu, soit en continu. Dans ce dernier cas, on alimente un réacteur ou plusieurs réacteurs en cascade simultanément avec la cyclohexanone et avec la solution brute d'acide percarboxylique. Le temps de séjour est compris entre 30 mn et 4 heures, suivant la température choisie pour la réaction.

Les exemples suivants illustrent de façon non limitative divers aspects de l'invention. Dans ces exemples, les teneurs en caprolactone et en cyclohexanone des solutions finales sont déterminées par chromatographie en phase gazeuse, tandis que l'oxygène peroxydique résiduaire est dosé chimiquement.

EXEMPLE 1

On prépare une solution d'acide perpropionique, selon la demande de brevet français n° 2 464 947 de la demanderesse, par action d'une solution aqueuse à 70% de peroxyde d'hydrogène sur l'acide propionique, en présence de 1% en poids d'acide borique, tout en éliminant continuellement l'eau du milieu réactionnel par entraînement azéotropique avec le dichloro-1,2-éthane.

La solution brute obtenue a la composition suivante:

| | |
|---|---|
| dichloro-1,2-éthane | 52,6% en poids |
| acide propionique | 33,0% en poids |
| acide perpropionique | 12,6% en poids |
| peroxyde d'hydrogène | 0,8% en poids |
| acide borique | 1,0% en poids |

Dans un réacteur en verre de 250 $cm^3$, muni d'un système d'agitation, surmonté d'un réfrigérant et équipé d'un système de contrôle de la température, on introduit en continu 175 g par heure de la solution brute d'acide perpropionique et 40 g par heure de cyclohexanone. La température du réacteur est maintenue à 60 °C et le temps de séjour des réactifs est d'environ 1 heure. On soutire en continu une solution contenant 13,2% en poids de caprolactone. Le taux de conversion de l'oxygène peroxydique est de 92% et la sélectivité en caprolactone est de 94%.

La solution de caprolactone obtenue est stable au stockage à température ambiante.

EXEMPLE 2 (Exemple comparatif)

On répète l'exemple 1, mais en utilisant une solution d'acide perpropionique obtenue par réaction du peroxyde d'hydrogène sur l'acide propionique en présence d'acide sulfurique comme catalyseur. Cette solution titre 0,195 mole d'acide perpropionique et 0,020 mole de peroxyde d'hydrogène pour 100 g. Elle contient aussi 0,05% en poids d'acide sulfurique.

Dans les mêmes conditions qu'à l'exemple 1, le taux de conversion de l'oxygène peroxydique est de 95% et la sélectivité en caprolactone de 10% seulement.

Après stockage de la solution finale pendant 24 heures à température ambiante, on ne retrouve plus de caprolactone à l'analyse.

EXEMPLE 3 (Exemple comparatif)

On répète l'exemple 2, en utilisant la même solution d'acide perpropionique obtenue par catalyse à l'acide sulfurique, mais après avoir neutralisé par une solution concentrée de soude les 0,05% en poids d'acide sulfurique.

Dans les conditions des exemples précédents, le taux de conversion de l'oxygène peroxydique est de 91% et la sélectivité en caprolactone de 81%.

Après stockage de la solution finale pendant 24 heures à la température ambiante, la sélectivité en caprolactone est tombée à 40%.

EXEMPLE 4

Dans un réacteur en verre maintenu à 50 °C et contenant 6 g de cyclohexanone et 50 g d'acide propionique, on introduit en 30 minutes 50 g d'une solution brute d'acide perpropionique, obtenue selon le brevet français n° 2 464 947 de la demanderesse, titrant 0,100 mole d'acide perpropionique et 0,004 mole de peroxyde d'hydrogène pour 100 g et contenant 0,5% en poids d'acide borique.

Après deux heures de réaction, on dose 0,048 mole de caprolactone. Le taux de conversion de l'oxygène peroxydique est de 94% et la sélectivité en caprolactone de 98%.

La stabilité au stockage à température ambiante de la solution de caprolactone obtenue est bonne.

EXEMPLE 5

On répète l'exemple 4 en remplaçant la solution brute d'acide perpropionique par une solution titrant 0,085 mole d'acide peracétique et 0,005 mole de peroxyde d'hydrogène pour 100 g et contenant 0,6% en poids d'acide borique.

Après deux heures de réaction, on dose 0,040 mole de caprolactone. Le taux de conversion de l'oxygène peroxydique est de 96% et la sélectivité en caprolactone de 92%.

La stabilité au stockage à température ambiante de la solution de caprolactone obtenue est bonne.

**Revendications**

1. Procédé de préparation de solutions stables d'ε-caprolactone par oxydation de la cyclohexanone au moyen d'un acide percarboxylique contenant de 2 à 4 atomes de carbone, caractérisé en ce que l'acide percarboxylique utilisé est sous forme d'une solution brute, telle qu'elle résulte de la réaction du peroxyde d'hydrogène sur l'acide carboxylique correspondant, en présence d'un acide borique comme catalyseur, avec élimination continue de l'eau par entraînement azéotropique.

2. Procédé selon la revendication 1, caractérisé en ce que la proportion d'acide percarboxylique dans la solution brute est de 5 à 40% en poids.

3. Procédé selon l'une des revendications 1 ou 2, caractérisé en ce que la solution brute d'acide percarboxylique contient un solvant organique miscible à l'acide percarboxylique et non susceptible de réagir avec lui.

4. Procédé selon la revendication 3, caractérisé en ce que le solvant organique est le solvant d'entraînement azéotropique qui a servi à l'élimination de l'eau au cours de la préparation de l'acide percarboxylique.

5. Procédé selon l'une des revendications 1 à 4, caractérisé en ce que la solution brute d'acide percarboxylique contient également jusqu'à 0,1 mole de peroxyde d'hydrogène pour 100 g de solution.

6. Procédé selon l'une des revendications 1 à 5, caractérisé en ce que la solution brute d'acide percarboxylique est une solution brute d'acide peracétique.

7. Procédé selon l'une des revendications 1 à 5, caractérisé en ce que la solution brute d'acide percarboxylique est une solution brute d'acide perpropionique.

8. Procédé selon l'une des revendications 1 à 7, caractérisé en ce que le rapport molaire de la cyclohexanone à l'acide percarboxylique est compris entre 1 et 5.

9. Procédé selon l'une des revendications 1 à 8, caractérisé en ce que la réaction d'oxydation de la cyclohexanone par la solution brute d'acide percarboxylique est conduite à une température comprise entre 20 et 120 °C.

**Claims**

1. Process for the preparation of stable solutions of ε-caprolactone by oxidation of cyclohexanone by means of a percarboxylic acid containing from 2 to 4 carbon atoms, characterised in that the percarboxylic acid used is in the form of a crude solution, such as results from the reaction of hydrogen peroxide with the corresponding carboxylic acid, in the presence of a boric acid as the catalyst, with continuous removal of the water by azeotropic entrainment.

2. Process according to Claim 1, characterised in that the proportion of percarboxylic acid in the crude solution is from 5 to 40% by weight.

3. Process according to one of Claims 1 or 2, characterised in that the crude solution of percarboxylic acid contains an organic solvent which is miscible with the percarboxylic acid and is not capable of reacting therewith.

4. Process according to Claim 3, characterised in that the organic solvent is the azeotropic entrainment solvent which has been used to remove the water during the preparation of the percarboxylic acid.

5. Process according to one of Claims 1 to 4, characterised in that the crude solution of percarboxylic acid also contains up to 0.1 mole of hydrogen peroxide per 100 g of solution.

6. Process according to one of Claims 1 to 5, characterised in that the crude solution of percarboxylic acid is a crude solution of peracetic acid.

7. Process according to one of Claims 1 to 5, characterised in that the crude solution of percarboxylic acid is a crude solution of perpropionic acid.

8. Process according to one of Claims 1 to 7, characterised in that the molar ratio of cyclohexanone to percarboxylic acid is between 1 and 5.

9. Process according to one of Claims 1 to 8, characterised in that the reaction of oxidising the cyclohexanone with the crude solution of percarboxylic acid is carried out at a temperature between 20 and 120 °C.

**Patentansprüche**

1. Verfahren zur Herstellung stabiler Lösungen von ε-Caprolacton durch Oxydation von Cyclohexanon mittels einer Percarbonsäure mit 2 bis 4 Kohlenstoffatomen, dadurch gekennzeichnet, dass die Percarbonsäure in Form der ungereinigten, bei der Reaktion von Wasserstoffperoxid mit der entsprechenden Carbonsäure entstehenden Lösung in Gegenwart einer Borsäure als Katalysator eingesetzt wird, wobei das Wasser kontinuierlich durch azeotropes Mitschleppen entfernt wird.

2. Verfahren nach Anspruch 1, dadurch gekennzeichnet, dass die Menge der Percarbonsäure in der ungereinigten Lösung zwischen 5 und 40 Gewichtsprozent beträgt.

3. Verfahren nach einem der Ansprüche 1 oder 2, dadurch gekennzeichnet, dass die ungereinigte Lösung der Percarbonsäure ein organisches Lösungsmittel enthält, das mit der Percarbonsäure mischbar ist und nicht mit ihr reagiert.

4. Verfahren nach Anspruch 3, dadurch gekennzeichnet, dass das organische Lösungsmittel das azeotrope Schleppmittel ist, das bei der Herstellung der Percarbonsäure zur Entfernung des Wassers gedient hat.

5. Verfahren nach einem der Ansprüche 1 bis 4, dadurch gekennzeichnet, dass die ungereinigte Percarbonsäurelösung bis zu 0,1 Mol Wasserstoffperoxid auf 100 g der Lösung enthält.

6. Verfahren nach einem der Ansprüche 1 bis 5, dadurch gekennzeichnet, dass die ungereinigte Lösung der Percarbonsäure eine ungereinigte Lösung von Peressigsäure ist.

7. Verfahren nach einem der Ansprüche 1 bis 5, dadurch gekennzeichnet, dass die ungereinigte Lösung der Percarbonsäure eine ungereinigte Lösung von Perpropionsäure ist.

8. Verfahren nach einem der Ansprüche 1 bis 7, dadurch gekennzeichnet, dass das Molverhältnis von Cyclohexanon zu der Percarbonsäure zwischen 1 und 5 liegt.

9. Verfahren nach einem der Ansprüche 1 bis 8, dadurch gekennzeichnet, dass die Oxydation des Cyclohexanons mit der ungereinigten Lösung der Percarbonsäure bei einer Temperatur zwischen 20 und 120 °C durchgeführt wird.